# EUROPEAN PATENT SPECIFICATION

(11) **EP 4 036 246 B1**
(45) Date of publication and mention of the grant of the patent: **09.04.2025**
(21) Application number: 20868918.2
(22) Date of filing: 24.09.2020
(51) Int. Cl.: C12Q 1/02, C12Q 1/68, G01N 1/30, G01N 33/48, G01N 33/50

(54) **A METHOD FOR TRANSPARENTIZING BIOLOGICAL TISSUE IN A LIVING STATE**
VERFAHREN ZUM OPTISCHEN KLÄREN VON BIOLOGISCHEM GEWEBE IM LEBENDEN ZUSTAND
PROCÉDÉ DE CLARIFICATION OPTIQUE DE TISSUS BIOLOGIQUES À L'ÉTAT VIVANT

(30) Priority: 24.09.2019 JP 2019172865
(43) Date of publication of application: 03.08.2022
(73) Proprietor: OSAKA UNIVERSITY, Suita-shi Osaka 565-0871 (JP)
(72) Inventor: MATSUSAKI, Michiya, Suita-shi, Osaka 565-0871 (JP)
(74) Representative: Hoffmann Eitle
(86) International application number: PCT/JP2020/036017
(87) International publication number: WO 2021/060373

(56) References cited:
- WO-A1-2014/115206
- WO-A1-2015/022883
- WO-A1-2018/030520
- WO-A1-2019/009300
- JP-A- 2003 066 035
- JP-A- 2003 066 035
- JP-A- 2016 538 569
- US-A1- 2017 130 254
- US-A1- 2019 128 785

## Description

### Technical Field

The present invention relates to a method for transparentizing biological tissue in a living state, the use of a composition and the use of a kit therefor.

### Background Art

In medical care and drug discovery research, it is important to accurately observe a three-dimensional structure of biological tissue, an organ, or a three-dimensional tissue model using a confocal laser scanning microscope or the like. However, since biological tissue is normally opaque and difficult for light to pass through, the limit of depth of observation is from 100 to 200 µm. Accordingly, various transparentizing methods have been reported in recent years. Many of these methods are techniques in which water is replaced with an organic molecule having a high refractive index to match the refractive index of a biomolecule, thereby increasing light transparency (see Patent Literature 1, for example). In addition, a method of lowering the refractive index of tissue by a technique of removing a lipid having a high refractive index has also been reported (see Patent Literature 2, for example).

However, since cells die after these treatments, it has been necessary to fix tissue with formaldehyde or the like, and there has been no report on a method that achieves tranparentization with the cells in a living state. In addition, a cell structure collapses after removal of lipid (cell membrane), which makes it impossible to observe fine molecules such as membrane proteins.

### Citation List

### Patent Literature

Patent Literature 1: International Publication No. WO2015/022883
Patent Literature 2: International Publication No. WO2017/188264

### Summary of Invention

### Technical Problem

There is a need to transparentize tissues and organs with the cells in a living state to enable a three-dimensional observation at a high depth.

### Solution to Problem

The inventors have made intensive studies to solve the above-described problem, and have found that biological tissue can be transparentized in a living state by applying a solution containing one or more compounds selected from the group consisting of a nucleotide, a nucleotide derivative, a macromolecule containing a nucleotide or a nucleotide derivative, and a nucleotide polymer to the biological tissue, thereby arriving at the completion of the present invention.

The present invention provides a method for transparentizing biological tissue in a living state, which comprises applying a solution to the biological tissue, the solution containing one or more compounds selected from the group consisting of a nucleotide, a nucleotide derivative, a macromolecule containing a nucleotide or a nucleotide derivative, and a nucleotide polymer.

Typically the compound is one or more compounds selected from the group consisting of AMP, CMP, TMP, ADP, and poly A.

Preferably the compound is one or more compounds selected from the group consisting of AMP, ADP, and poly A.

Furthermore the present invention provides the use of a composition for transparentizing biological tissue in a living state, which comprises one or more compounds selected from the group consisting of a nucleotide, a nucleotide derivative, a macromolecule containing a nucleotide or a nucleotide derivative, and a nucleotide polymer.

Typically the composition used comprises one or more compounds selected from the group consisting of AMP, CMP, TMP, ADP, and poly A.

Preferably the composition used comprises one or more compounds selected from the group consisting of AMP, ADP, and poly A.

Furthermore the present invention provides the use of a kit for transparentizing biological tissue in a living state, which comprises one or more compounds selected from the group consisting of a nucleotide, a nucleotide derivative, a macromolecule containing a nucleotide or a nucleotide derivative, and a nucleotide polymer.

Typically the kit used comprises one or more compounds selected from the group consisting of AMP, CMP, TMP, ADP, and poly A.

Preferably the kit used comprises one or more compounds selected from the group consisting of AMP, ADP, and poly A.

Furthermore the present invention provides a method for observing biological tissue, which comprises transparentizing biological tissue in a living state using the method according to any one of (1) to (3), the composition according to any one of (4) to (6), or the kit according to any one of (7) to (9) to observe the inside of the biological tissue.

### Effects of Invention

Since according to the present invention biological tissue can be transparentized in a living state, state of a deep part of the biological tissue can be observed as it is. The state of a blood vessel or lymph vessel, gene expression, localization of a substance and so on in the deep part of the biological tissue can be observed over time. In addition, since not only the surface but also the deep part of a graft can be observed, the graft can be accurately evaluated. Therefore, it can be said that the significance of the ability to transparentize biological tissue in a living state is extremely great in the development of pharmaceuticals, the medical and pharmaceutical science, and the like. Further, the compound used in the present invention is commercially available and is low cost.

### Brief Description of Drawings

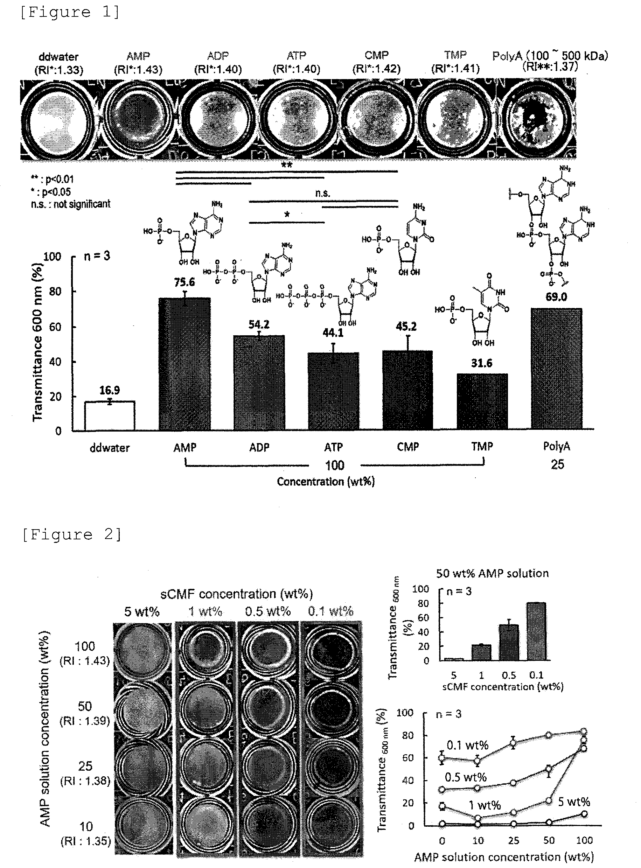

[Figure 1] The upper panel of Figure 1 is a photograph showing a transparentizing effect of AMP, ADP, ATP, CMP, TMP, and poly A on a collagen solution. The lower panel of Figure 1 is a graph showing light transmittance of the collagen solution treated with AMP, ADP, ATP, CMP, TMP, and poly A. * indicates 100 wt%; ** indicates 25 wt%.
[Figure 2] Figure 2 shows a photograph and graph showing results of examining the effect of an AMP concentration and a collagen concentration on the transparentization.

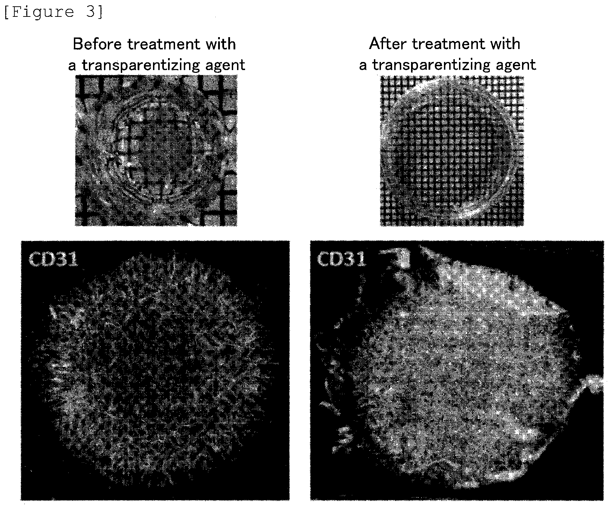

[Figure 3] Figure 3 shows a photograph showing the appearance of an artificial three-dimensional tissue model before and after AMP treatment (upper left panel before the treatment, upper right panel after the treatment), and a confocal laser scanning microscope photograph for an observation of a blood vessel inside tissue with CD31 antibody-staining (lower left panel before the treatment, lower right panel after the treatment). All the photographs in Figure 3 are taken in a direction perpendicular to the dish.

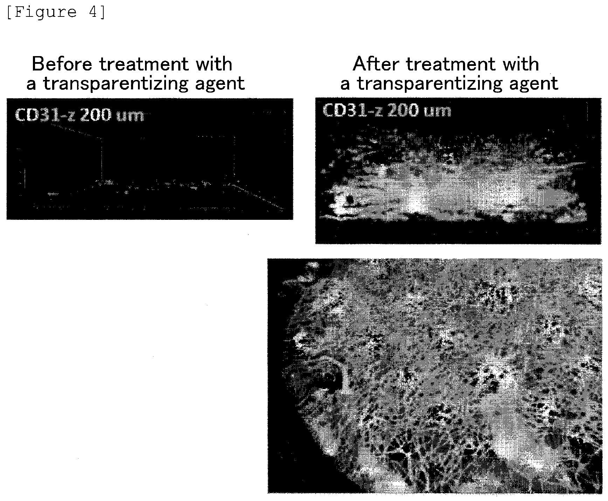

[Figure 4] Figure 4 shows a confocal laser scanning microscope photograph for an observation of a blood vessel inside tissue of an artificial three-dimensional tissue model before and after AMP treatment (upper left panel before the treatment, upper right panel after the treatment). The blood vessel was stained with a CD31 antibody. These photographs are taken in a direction horizontal to the dish. The lower right panel of Figure 4 is a confocal laser microscope photograph of the entire vascular network after a transparentization treatment.

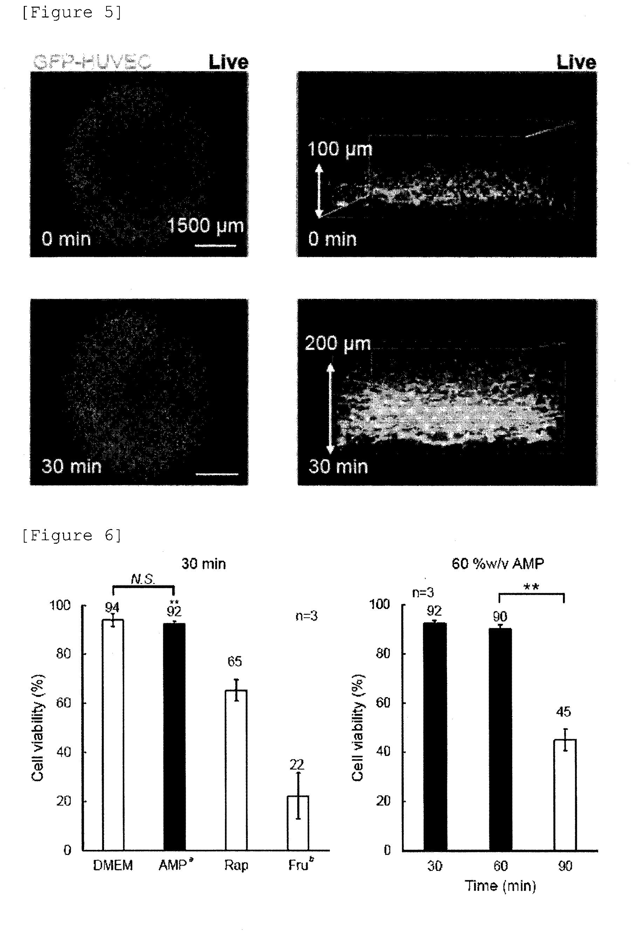

[Figure 5] The upper part of Figure 5 shows images immediately before the treatment (0 minutes), in which the left panel is an image observed in a direction perpendicular to the dish, and the right panel is an image observed in a direction horizontal to the dish. The lower part of Figure 5 shows images immediately after the treatment (30 minutes), in which the left panel is an image observed in a direction perpendicular to the dish, and the right panel is an image observed in a direction horizontal to the dish.
[Figure 6] The left panel of Figure 6 shows the cell survival rate after 30 minutes of transparentization treatment with AMP, RapiClear 1.52, and fructose. The right panel of Figure 6 shows the relationship between the time of transparentization treatment with AMP and cell survival rate. In the figure, Rap represents RapiClear 1.52 and Fru represents fructose. ** indicates p < 0.01; N.S. indicates non-significance.

### Mode for Carrying Out the Invention

In one aspect, the present invention provides a method for transparentizing biological tissue in a living state, which comprises applying a solution to the biological tissue, the solution containing one or more compounds selected from the group consisting of a nucleotide, a nucleotide derivative, a macromolecule containing a nucleotide or a nucleotide derivative, and a nucleotide polymer.

Any nucleotide, any nucleotide derivative, any macromolecule containing a nucleotide or a nucleotide derivative, and any nucleotide polymer that have transparentizing ability may be used in the present invention. A nucleotide is a compound having a nucleoside bonded to a phosphate group. A nucleoside is a compound having a base bonded to a sugar. Typical examples of the base include, but are not limited to, purine bases such as adenine and guanine and pyrimidine bases such as thymine, cytosine, and uracil. Examples of the base also include nicotinamide and dimethylisoalloxazine. Typical examples of the sugar include, but are not limited to, ribose and deoxyribose. A nucleotide containing ribose as a sugar is referred to as a ribonucleoside, and a ribonucleoside having a phosphate group bonded thereto is referred to as a ribonucleotide. A nucleoside containing deoxyribose as a sugar is referred to as a deoxyribonucleoside, and a deoxyribonucleoside having a phosphate group bonded thereto is referred to as a deoxyribonucleotide.

The nucleotide polymer refers to a compound in which two or more nucleotides are bonded through phosphodiester bond between the phosphate group of one nucleotide and the sugar of another nucleotide. Typical examples of the nucleotide polymer include polymers of ribonucleotides and polymers of deoxyribonucleotides. In the present invention, the nucleotide polymer may be a polymer of ribonucleotides, a polymer of deoxyribonucleotides, or a polymer of ribonucleotides and deoxyribonucleotides.

A typical nucleotide, nucleotide derivative, macromolecule containing a nucleotide or a nucleotide derivative, and nucleotide polymer used in the present invention are described below.

Examples of the ribonucleotide include, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), uridine monophosphate (UMP), uridine diphosphate (UDP), uridine triphosphate (UTP), 5-methyluridine monophosphate (TMP), 5-methyluridine diphosphate (TDP), 5-methyluridine triphosphate (TTP), cytidine monophosphate (CMP), cytidine diphosphate (CDP), cytidine triphosphate (CTP), inosine monophosphate (IMP), inosine diphosphate (IDP), and inosine triphosphate (ITP). Preferred examples of the ribonucleotide include AMP, ADP, ATP, CMP, GMP, IMP, TMP, and UMP, and more preferred examples of the ribonucleotide include AMP, ADP, CMP, and TMP. Still more preferred examples of the ribonucleotide include AMP and ADP.

Examples of the deoxyribonucleotide include, but are not limited to, deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), thymidine monophosphate (dTMP), thymidine diphosphate (dTDP), thymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP), and deoxycytidine triphosphate (dCTP). Preferred examples of the deoxyribonucleotide include dAMP, dGMP, dTMP, dUMP, and dCMP. More preferred examples of the deoxyribonucleotide include dTMP.

A variety of nucleotide derivatives are known. Examples of derivatization include, but are not limited to, alkylation, esterification, halogenation, addition of phosphoric acid, addition of an amino acid or peptide, and addition of a heterocycle. The nucleotide may have a ring formed in its molecule. Typical examples of the cyclized nucleotide include, but are not limited to, cyclic AMP (cAMP). A nucleotide from which the nucleotide derivative is derived may be a ribonucleotide or a deoxyribonucleotide.

The macromolecule containing a nucleotide or a nucleotide derivative refers to a macromolecule having a macromolecule backbone to which a nucleotide(s) or a nucleotide derivative(s) is (are) bonded. For example, an appropriate number of nucleotides or nucleotide derivatives may be bonded, for example, through a functional group of a polysaccharide or polypeptide. For example, a part of sugar residues of the polysaccharide or of amino acid residues of the polypeptide may be replaced with a nucleotide or nucleotide derivative. The nucleotide in such a macromolecule may be a ribonucleotide, a deoxyribonucleotide, a ribonucleotide and deoxyribonucleotide, or a derivative thereof.

Nucleotides constituting the nucleotide polymer may be ribonucleotides, deoxyribonucleotides, or ribonucleotides and deoxyribonucleotides. A polymer of ribonucleotides may be formed from a single kind of ribonucleotide or may be formed from two or more kinds of ribonucleotides. Preferred examples of the ribonucleotide polymer include, but are not limited to, poly A that is polymerized AMP. A molecular weight of the ribonucleotide polymer is not particularly limited, and may be, for example, from about 100 to several hundred kDa. A polymer of deoxyribonucleotides may be formed from one kind of deoxyribonucleotide or may be formed from two or more kinds of deoxyribonucleotides. A molecular weight of the deoxyribonucleotide polymer is not particularly limited, and may be, for example, from about 100 to several hundred kDa.

A part or all of the nucleotides constituting the polymer may be a nucleotide derivative.

A variety of nucleotides are commercially available and can be used in the present invention. Methods for chemical synthesis and enzymatic synthesis of nucleotides are also known. A variety of nucleotide polymers are commercially available and can be used. Methods for synthesizing polymers of nucleotides are also known, including solid-phase synthesis and liquid-phase synthesis. Typical examples of the solid-phase synthesis include the phosphoramidite method. Methods for synthesizing nucleotide derivatives are also known. Methods for synthesizing macromolecules containing nucleotides or nucleotide derivatives are also known. Those skilled in the art can obtain a desired nucleotide, nucleotide derivative, macromolecule containing a nucleotide or a nucleotide derivative, and nucleotide polymer using a known synthesis method.

It is preferable that the nucleotide, nucleotide derivative, macromolecule containing a nucleotide or nucleotide derivative, and nucleotide polymer that are used in the present invention have low or no toxicity to cells.

In the present invention, one compound or two or more compounds selected from the group consisting of the nucleotide, the nucleotide derivative, the macromolecule containing a nucleotide or a nucleotide derivative, and a nucleotide polymer may be used. For example, a mixture of AMP and poly A may be used.

In the method of the present invention, a solution containing the above-described compound is applied to biological tissue. The solution can be prepared by dissolving the above-described compound in water or a buffer solution. The buffer solution is known and can be selected as appropriate. Examples of the buffer solution include, but are not limited to, Tris-HCl buffer, phosphate buffer, and phosphate-buffered saline.

A concentration of the compound in the solution is preferably such that the solution has a refractive index of about 1.35 or more, preferably about 1.4 or more. Since the refractive index varies from compound to compound, the concentration to obtain a desired refractive index also varies from compound to compound. Selection of the compound and adjustment of its concentration are within the skill of those skilled in the art. Means and methods for measuring a refractive index are known.

The solution above described may be applied to biological tissue in any manner as long as the compound can surround the biological tissue to transparentize the biological tissue. The solution may be poured into a container containing biological tissue attached thereto so that the entire of the tissue is submerged, or biological tissue may be suspended in the solution in a suitable container. In an embodiment, medium is removed from biological tissue being cultured, for example, in a dish or well or on a membrane, and then the solution is added to cover the biological tissue. The resultant is allowed to stand until transparentization as desired is attained, and then the solution is removed to observe the tissue. In another embodiment, biological tissue is isolated from culture medium and transferred into a container containing the solution. The resultant is allowed to stand until transparentization as desired is attained, and then the solution is removed to observe the tissue. The applying manner is not limited to the embodiments described above.

Biological tissue encompasses tissue derived from any organism, and examples of the biological tissue include, but are not limited to, those derived from animals, plants, fish, amphibians, reptiles, insects, and microorganisms. Biological tissue may be derived from a mammal such as a human, a monkey, a dog, a cat, a pig, a cow, a rabbit, a rat, a mouse, or a guinea pig, or may be derived from an avian such as a chicken.

Biological tissue may be derived from any site of an organism. When the organism is an animal, the biological tissue may be derived from, for example, a heart, lung, liver, kidney, stomach, intestine, pancreas, gallbladder, reproductive organ, brain, skin, muscle, or others. Biological tissue may be biopsy specimen, tissue from surgery, tissue from dissection, or the like. Biological tissue may be artificially prepared. Examples of the artificial biological tissue include, but are not limited to, those prepared by a LbL method that is described, for example, in Japanese Patent Laid-Open No. 2012-115254. Biological tissue may be obtained by a known culture method. Biological tissue may be a spheroid. Biological tissue may be a graft. Biological tissue may be a normal tissue or a tissue having a disease, for example, a cancer tissue. The term "biological tissue" as used herein encompasses cells, cell masses, tissues, apparatus, organs, and individual orgamisns (excluding living human individuals). Biological tissue may be a decellularized tissue.

The term "a living state" means that survival rate of cells in biological tissue subjected to the transparentization treatment using the above-described compound is about 30% or more, preferably about 40% or more, more preferably about 50% or more, still more preferably about 60% or more, most preferably about 70% or more, for example, about 80% or more. The cell survival rate can be measured by a known method such as trypan blue staining.

The transparentization may be carried out so that, for example, the transmittance of light at 600 nm is about 30% or more, preferably about 40% or more, more preferably about 50% or more, still more preferably about 60% or more, most preferably about 70% or more, for example, about 80% or more, at a desired site in the tissue. The transmittance is expressed in terms of a ratio of transmitted light to incident light. Methods and means for measuring transmittance are known to those skilled in the art. Those skilled in the art can select and determine conditions for achieving transparentization, including selection of a compound, concentration of a compound, transparentization treatment time, temperature, pH, and presence or absence of shaking, in consideration of type of biological tissue and a desired transparentization depth, for example.

The composition used for transparentizing biological tissue in a living state comprises one or more compounds selected from the group consisting of a nucleotide, a nucleotide derivative, a macromolecule containing a nucleotide or a nucleotide derivative, and a nucleotide polymer.

Form of the composition is not particularly limited but is usually a solution containing the above-described compound. The solution is preferably obtained by dissolving the compound in an aqueous medium. Examples of the aqueous medium include water and a buffer solution. The buffer solution is as described above. The concentration of the compound in the solution is as described above. Alternatively, the composition may be solid (e.g., powder, granules, pellets, etc.) or semi-solid (e.g., paste, gel, etc.). The composition of the present invention may be diluted with or dissolved in an aqueous medium prior to use. The composition of the present invention may further contain any compound, e.g., components such as an organic material and an inorganic material. The composition can be applied to biological tissue to transparentize the biological tissue in a living state. The application is as described above.

The kit used for transparentizing biological tissue in a living state comprises one or more compounds selected from the group consisting of a nucleotide, a nucleotide derivative, a macromolecule containing a nucleotide or a nucleotide derivative, and a nucleotide polymer.

The kit comprises the above-described compound as an essential component. Specifically, the kit of the present invention may include a container containing the above-described compound in the form of a solution in an aqueous medium, or a container containing the above-described compound in the form of, for example, powder, granules, pellets, paste, or gel. Form of the container is not particularly limited and may be, for example, a bottle, a bag, or a tube. Typically, an instruction for use is attached to the kit of the present invention.

In another aspect, the present invention provides a method for observing biological tissue, which comprises transparentizing the biological tissue in a living state using the method, composition, or kit of the present invention to observe the inside of the biological tissue.

In the observation method of the present invention, biological tissue transparentized in a living state that is obtained using the method, composition, or kit of the present invention is observed with the naked eye or using an instrument such as a microscope. Means and methods for observation are known to those skilled in the art and can be selected and used as appropriate according to the type and size of the tissue. For example, after a transparentized sample is immunostained with a fluorescence-labeled antibody, a specific substance or site in biological tissue may be detected by a fluorescence microscope. According to the observation method of the present invention, it is possible to see state of a deep part of biological tissue, for example, development of a vascular system, production of a substance, gene expression, and a lesion.

Also disclosed is a method for producing biological tissue transparentized in a living state, which comprises using the method, composition, or kit described herein.

As described above, the method of the present invention is preferably applicable to living biological tissue, however are of course applicable to non-living biological tissue as well.

Unless otherwise specified, the meanings of terms as used herein are understood to be those normally understood in the fields of chemistry, biology, pharmacy, medicine, etc.

Hereinafter, the present invention will be described in more detail and more specifically with reference to examples, but these examples do not limit the scope of the present invention.

### Example 1

### Example 1. Transparentizing ability of nucleotide and nucleotide polymer in collagen model system

Since collagen is most abundant in biological tissue, a collagen solution was used as a model of the biological tissue to screen various compounds for their transparentizing ability. The outline of the screening experiment is as follows.

Aqueous solutions of test compounds were added to multi-well plates containing aqueous solutions of sCMF (sonicated collagen microfiber) and allowed to stand at room temperature for a certain period of time, and subsequently, apparent transparency and transmittance at 600 mn were measured.

As a result, some compounds having an excellent transparentizing ability were found among nucleotides and ribonucleotide polymers. In particular, AMP, ADP, ATP, CMP, TMP and poly A were found to have a high transparentizing ability. Among these compounds, the cell survival rate was low for ATP, however it was considered that the cell survival rate would be improved by adjusting the transparentization conditions such as the transparentization treatment time and temperature, and the concentrations of these compounds.

Transparentization experiments using AMP, ADP, ATP, CMP, TMP, and poly A are described below.

100 µL of 100 wt% AMP aqueous solution (refractive index: 1.43), 100 wt% CMP aqueous solution (refractive index: 1.42), 100 wt% TMP aqueous solution (refractive index: 1.41) or 25 wt% poly A aqueous solution (refractive index: 1.37) were added. The molecular weight of poly A was 100 to 500 kDa. Distilled water was added for a control. The resultant was allowed to stand at room temperature for 2 hours, and then the absorbance at 600 nm was measured with a plate reader (Synergy HTX). The obtained absorbance was substituted into an equation according to the Lambert-Beer formula to calculate the transmittance.

The upper panel of Figure 1 shows the plate from the transparentization treatment for 2 hours, and the lower panel of Figure 1 shows the transmittance. The transmittance when AMP was used (75.6%) was the highest, and the transmittance when poly A was used (69.0%) was the second highest. The transmittances when CMP and TMP were used were 45.2% and 31.6%, respectively.

### Example 2

### Example 2. Effect of transparentation treatment on cell survival rate

The cell survival rates in the transparentization using AMP, ADP, ATP, CMP, TMP, fructose, glucose, trehalose, sucrose, antipyrine, RapiClear 1.52, and poly A were examined. 1.0 x 10⁵ human skin fibroblasts were seeded in a 24-well plate and cultured in DMEM medium. The next day, after washing with PBS, 100 µL of 100 wt% AMP aqueous solution, 100 wt% ADP aqueous solution, 100 wt% ATP aqueous solution, 100 wt% CMP aqueous solution, 100 wt% TMP aqueous solution, 100 wt% fructose aqueous solution, 100 wt% glucose aqueous solution, 100 wt% trehalose aqueous solution, 100 wt% sucrose aqueous solution, 100 wt% antipyrine aqueous solution, RapiClear 1.52 aqueous solution, or 25 wt% poly A aqueous solution was added, and the resultant was incubated at 37°C for 30 minutes. Distilled water was added for a control. After the incubation, 0.1% trypsin was directly added, and the resultant was incubated for 5 minutes. Cell dissociation was confirmed, 3 mL of DMEM medium was added, and then the resultant was collected into a 15-mL centrifuge tube, and centrifuged (1000 rpm, 5 minutes, 20°C). The supernatant was aspirated and 100 µL of DMEM medium was added. 10 µL was separated from the resultant and placed in an Eppendorf tube, and 10 µL of trypan blue solution was added thereto. Then, 10 µL was separated and the number of cells was counted with an automated cell counter. The results are shown in Table 1.

**[Table 1]**

| Table 1 | Cell survival rate after transparentization treatment (n = 3) |
|---|---|
| Treatment | Survival rate (%) |
| AMP | 92^{*} |
| ADP | 87^{*} |
| ATP | 26 |
| CMP | 53 |
| TMP | 42 |
| Fructose | 6 |
| Glucose | 16 |
| Trehalose | 25 |
| Sucrose | 14 |
| Antipyrine | 33 |
| RapiClear 1.52 | 29 |
| Distilled water | 96 |

| | |
|---|---|
| ^{*}p<0.05 | |

It was confirmed that a significant number of cells survived after the transparentization treatment. The survival rate in the transparentization treatment with AMP was the highest. The survival rate in the transparentization treatment with ADP was the next highest. The cell survival rate was higher in the case of treatment with CMP and TMP as well than in the case of treatment with RapiClear 1.52 and antipyrine, which are known transparentizing agents. When poly A was used, it was impossible to perform trypan blue staining due to the high viscosity of poly A; however, it was deemed that the transparentization treatment with poly A had little effect on the survival rate (data not shown).

### Example 3

### Example 3. Effect of AMP concentration and collagen concentration on the transparentization

Effects of an AMP concentration and a collagen concentration on the transparentization were examined using a collagen solution as a model of biological tissue.

100 µL of 0.1 wt%, 0.5 wt%, 1 wt%, and 5 wt% sCMF aqueous solutions were placed in a 96-well plate, and 100 µL of 10 wt% (refractive index: 1.35), 25 wt% (refractive index: 1.38), 50 wt% (refractive index: 1.39), and 100 wt% (refractive index: 1.43) AMP aqueous solutions were added. After allowing the obtained samples to stand at room temperature for 2 hours, an absorbance at 600 nm was measured with a plate reader (Synergy HTX). The obtained absorbance was substituted into an equation according to the Lambert-Beer formula to calculate the transmittance.

The left panel of Figure 1 shows the plate from the transparentization treatment for 2 hours. The upper right panel of Figure 1 shows the relationship between the sCMF concentration and the transmittance when 50 wt% AMP was used. The effects of the AMP concentration and the sCMF concentration on transmittance are shown in Figure 2.

It was found that as the collagen concentration was lower, the transmittance was higher, and that as the AMP concentration was higher, the transmittance was higher. From these results, it was deemed that the concentration of the compound used for the transparentization can be adjusted according to, for example, thickness of biological tissue (a desired transparentization depth) and cell density of biological tissue.

### Example 4

### Example 4. Transparentization test (1) on three-dimensional tissue model

The experimental procedure is as follows:
(1) A solution containing fibrinogen at a concentration of 2 mg/100 µL, CMF at a concentration of 6 mg/100 µL, and thrombin at a concentration of 1 unit/100 µL using DMEM was prepared.
(2) 1.0 x 10⁵ of human skin fibroblasts, 5.0 x 10⁴ of human umbilical vein endothelial cells, 18 µL of a mixed medium of DMEM and EGM-2, and 2 µL of a thrombin solution were placed in an Eppendorf tube to prepare a cell suspension.
(3) The fibrinogen solution prepared in (1) was combined with a CMF solution, and 40 µL from 200 µL in total of the resulting solution was taken out and added to the cell suspension prepared in (2).
(4) The cell suspension prepared in (3) was placed in a glass bottom dish to prepare a 30-µL drop.
(5) After the drop was incubated for 2 hours, 2 mL of a mixed medium of DMEM and EGM-2 was added.
(6) The drop was cultured at 37°C for 1 week, washed with PBS, and then fixed with paraformaldehyde for 15 minutes.
(7) The fixed tissue was subjected to CD31 immunostaining and DAPI staining.
(8) A transparentization treatment was performed with 100 wt% AMP at 37°C for 30 minutes.
(9) The tissue was observed with a confocal laser scanning microscope (Olympus FV3000).

The upper part of Figure 3 shows photographs of the tissue (in a direction perpendicular to the dish) before and after the transparentization. It was confirmed with the naked eye that the tissue was completely transparentized. The lower part of Figure 3 shows images of immunostained blood vessels inside the tissue (in a direction perpendicular to the dish) before and after the transparentization treatment. The blood vessels in the deep part of the tissue were unclear before the transparentization treatment (left), whereas the blood vessels in the deep part of the tissue from the transparentization treatment were clearly observed (right).

The upper part of Figure 4 shows images of immunostained blood vessels inside the tissue (in a direction horizontal to the dish) before and after the transparentization treatment. Almost no blood vessels were observed before the transparentization treatment, whereas the blood vessels in the deep part were clearly observed by the transparentization treatment. The lower right panel of Figure 4 is a confocal laser microscope photograph of the entire vascular network after a transparentization treatment. From this photograph, it was confirmed that a vascular netwok was formed throughout a large space of several millimeters.

### Example 5

### Example 5. Transparentization test (2) on three-dimensional tissue model

A transparentization was performed on a living three-dimensional tissue model. 60 wt% AMP was used as a transparentizing agent, and RapiClear 1.52 and 61 wt% fructose were used for comparison. The experimental procedure was the same as that of Example 4, except that the paraformaldehyde fixation described in step (6) was not performed.

Figure 5 shows images of immunostained blood vessels inside the tissue before and after the transparentization treatment with AMP. The upper part of Figure 5 is images immediately before the treatment (0 minutes), in which the left panel is an image observed in a direction perpendicular to the dish, and the right panel is an image observed in a direction horizontal to the dish. The lower part of Figure 5 is images immediately after the treatment (30 minutes), in which the left panel is an image observed in a direction perpendicular to the dish, and the right panel is an image observed in a direction horizontal to the dish. By the transparentization treatment with AMP, the central portion of the tissue was seen so that the deep part up to 200 µm could be observed.

Further after 30, 60, and 90 minutes of the transparentization treatment in the same experimental system as described above, the tissue was disintegrated with proteinase K and collagenase (1:1) and subjected to trypan blue staining to count the number of viable cells. The results are shown in Figure 6. The left panel of Figure 6 shows the cell survive rate after 30 minutes of transparentization treatment with AMP, RapiClear 1.52, and fructose. The transparentization treatment with AMP had no significant effect on the cell survival rate (a cell survival rate of 92%). On the other hand, the transparentization treatment with RapiClear 1.52 and fructose clearly reduced the cell survival rate (65% and 22%, respectively). The right panel of Figure 6 shows the relationship between the time of transparentization treatment with AMP and cell survival rate. The cell survival rate was as high as 92% and 90% after the 30-minute and 60-minute transparentization treatments, respectively. It was confirmed that a significant number of cells survived even after the 90-minute transparentization treatment (a cell survival rate of 45%) .

### Industrial Applicability

The present invention is applicable in the development of pharmaceuticals, the medical and biological science, or the like.

## Claims

1. A method for transparentizing biological tissue in a living state, which comprises applying a solution to the biological tissue, the solution containing one or more compounds selected from the group consisting of a nucleotide, a nucleotide derivative, a macromolecule containing a nucleotide or a nucleotide derivative, and a nucleotide polymer.

2. The method according to claim 1, wherein the compound is one or more compounds selected from the group consisting of AMP, CMP, TMP, ADP, and poly A.

3. The method according to claim 2, wherein the compound is one or more compounds selected from the group consisting of AMP, ADP, and poly A.

4. Use of a composition for transparentizing biological tissue in a living state, which comprises one or more compounds selected from the group consisting of a nucleotide, a nucleotide derivative, a macromolecule containing a nucleotide or a nucleotide derivative, and a nucleotide polymer.

5. The use of a composition according to claim 4, wherein the compound is one or more compounds selected from the group consisting of AMP, CMP, TMP, ADP, and poly A.

6. The use of a composition according to claim 5, wherein the compound is one or more compounds selected from the group consisting of AMP, ADP, and poly A.

7. Use of a kit for transparentizing biological tissue in a living state, which comprises one or more compounds selected from the group consisting of a nucleotide, a nucleotide derivative, a macromolecule containing a nucleotide or a nucleotide derivative, and a nucleotide polymer.

8. The use of a kit according to claim 7, wherein the compound is one or more compounds selected from the group consisting of AMP, CMP, TMP, ADP, and poly A.

9. The use of a kit according to claim 8, wherein the compound is one or more compounds selected from the group consisting of AMP, ADP, and poly A.

10. A method for observing biological tissue, which comprises transparentizing the biological tissue in a living state using the method according to any one of claims 1 to 3, the use of a composition according to any one of claims 4 to 6, or the use of a kit according to any one of claims 7 to 9 to observe the inside of the biological tissue.

## Patentansprüche

1. Verfahren zum Transparentmachen von biologischem Gewebe im lebenden Zustand, dass das Auftragen einer Lösung auf das biologische Gewebe umfasst, wobei die Lösung eine oder mehrere Verbindungen enthält, ausgewählt aus der Gruppe bestehend aus einem Nukleotid, einem Nukleotidderivat, einem Makromolekül bestehend aus einem Nukleotid oder einem Nukleotidderivat, und einem Nukleotidpolymer.

2. Verfahren gemäß Anspruch 1, wobei die Verbindung eine oder mehrere Verbindungen ist, ausgewählt aus der Gruppe bestehend aus AMP, CMP, TMP, ADP und Poly A.

3. Verfahren gemäß Anspruch 2, wobei die Verbindung eine oder mehrere Verbindungen ist, ausgewählt aus der Gruppe, bestehend aus AMP, ADP und Poly A.

4. Verwendung einer Zusammensetzung zum Transparentmachen von biologischem Gewebe im lebenden Zustand, die eine oder mehrere Verbindungen umfasst, ausgewählt aus der Gruppe bestehend aus einem Nukleotid, einem Nukleotidderivat, einem Makromolekül bestehend aus einem Nukleotid oder einem Nukleotidderivat, und einem Nukleotidpolymer.

5. Verwendung einer Zusammensetzung gemäß Anspruch 4, wobei die Verbindung eine oder mehrere Verbindungen ist, ausgewählt aus der Gruppe bestehend aus AMP, CMP, TMP, ADP und Poly A.

6. Verwendung einer Zusammensetzung gemäß Anspruch 5, wobei die Verbindung eine oder mehrere Verbindungen ist, ausgewählt aus der Gruppe, bestehend aus AMP, ADP und Poly A.

7. Verwendung eines Kits zum Transparentmachen von biologischem Gewebe im lebenden Zustand, das eine oder mehrere Verbindungen umfasst, ausgewählt aus der Gruppe bestehend aus einem Nukleotid, einem Nukleotidderivat, einem Makromolekül bestehend aus einem Nukleotid oder einem Nukleotidderivat, und einem Nukleotidpolymer.

8. Verwendung eines Kits gemäß Anspruch 7, wobei die Verbindung eine oder mehrere Verbindungen ist, ausgewählt aus der Gruppe bestehend aus AMP, CMP, TMP, ADP und Poly A.

9. Verwendung eines Kits gemäß Anspruch 8, wobei die Verbindung eine oder mehrere Verbindungen ist, ausgewählt aus der Gruppe, bestehend aus AMP, ADP und Poly A.

10. Verfahren zur Beobachtung von biologischem Gewebe, dass das Transparentmachen des biologischen Gewebes in einem lebenden Zustand unter Verwendung des Verfahrens gemäß irgendeinem der Ansprüche 1 bis 3 umfasst, die Verwendung einer Zusammensetzung gemäß irgendeinem der Ansprüche 4 bis 6, oder die Verwendung eines Kits gemäß irgendeinem der Ansprüche 7 bis 9, um das Innere des biologischen Gewebes zu beobachten.

## Revendications

1. Procédé de clarification d'un tissu biologique dans un état vivant, qui comprend l'application d'une solution au tissu biologique, la solution contenant un ou plusieurs composés choisis dans le groupe consistant en nucléotide, dérivé nucléotidique, macromolécule contenant un nucléotide ou un dérivé nucléotidique, et polymère nucléotidique.

2. Procédé selon la revendication 1, dans lequel le composé est un ou plusieurs composés choisis dans le groupe consistant en AMP, CMP, TMP, ADP, et poly A.

3. Procédé selon la revendication 2, dans lequel le composé est un ou plusieurs composés choisis dans le groupe consistant en AMP, ADP, et poly A.

4. Utilisation d'une composition de clarification d'un tissu biologique dans un état vivant, qui comprend un ou plusieurs composés choisis dans le groupe consistant en nucléotide, dérivé nucléotidique, macromolécule contenant un nucléotide ou un dérivé nucléotidique, et polymère nucléotidique.

5. Utilisation d'une composition selon la revendication 4, dans laquelle le composé est un ou plusieurs composés choisis dans le groupe consistant en AMP, CMP, TMP, ADP, et poly A.

6. Utilisation d'une composition selon la revendication 5, dans laquelle le composé est un ou plusieurs composés choisis dans le groupe consistant en AMP, ADP, et poly A.

7. Utilisation d'un kit de clarification d'un tissu biologique dans un état vivant, qui comprend un ou plusieurs composés choisis dans le groupe consistant en nucléotide, dérivé nucléotidique, macromolécule contenant un nucléotide ou un dérivé nucléotidique, et polymère nucléotidique.

8. Utilisation d'un kit selon la revendication 7, dans laquelle le composé est un ou plusieurs composés choisis dans le groupe consistant en AMP, CMP, TMP, ADP, et poly A.

9. Utilisation d'un kit selon la revendication 8, dans laquelle le composé est un ou plusieurs composés choisis dans le groupe consistant en AMP, ADP, et poly A.

10. Procédé permettant d'observer un tissu biologique, qui comprend la clarification du tissu biologique dans un état vivant en utilisant le procédé selon l'une quelconque des revendications 1 à 3, l'utilisation d'une composition selon l'une quelconque des revendications 4 à 6, ou l'utilisation d'un kit selon l'une quelconque des revendications 7 à 9 pour observer l'intérieur du tissu biologique.
